# EUROPEAN PATENT SPECIFICATION

(11) **EP 0 424 466 B1**
(45) Date of publication and mention of the grant of the patent: **17.04.1996**
(21) Application number: 89908938.7
(22) Date of filing: 07.07.1989
(51) Int. Cl.: C07D 213/09

(54) **PROCESS FOR SELECTIVE PRODUCTION OF 3-METHYLPYRIDINE**
VERFAHREN ZUR SELEKTIVEN PRODUKTION VON 3-METHYLPYRIDIN
PROCEDE DE PRODUCTION SELECTIVE DE 3-METHYLPYRIDINE

(30) Priority: 11.07.1988 US 217686
(43) Date of publication of application: 02.05.1991
(73) Proprietor: REILLY INDUSTRIES, INC., Indianapolis Indiana 46204 (US)
(72) Inventor: GOE, Gerald, L., Greenwood, IN 46142 (US); DAVIS, Robert, D., Indianapolis, IN 46222 (US)
(74) Representative: Bannerman, David Gardner
(86) International application number: US8902971
(87) International publication number: WO9000547

(56) References cited:
- EP-A- 0 061 982
- EP-A- 0 177 272
- DE-A- 2 519 529

## Description

### Background of the Invention

The object of this invention is to provide a new, economical process for the selective production at high yield of 3-methylpyridine (also called beta-picoline) through the catalytic cyclization of a pentanediamine derivative, namely 2-methyl-1,5-pentanediamine. Another object is the similar conversion of a mixture of this acyclic compound and a piperidine derivative, namely 3-methylpiperidine (also called beta-pipecoline), to the same desired 3-methylpyridine product.

As background, the value of this invention is enhanced by the fact that the starting materials are readily available often as by-products from the manufacture of other large-volume products. For instance, in the manufacture of adiponitrile which is an important intermediate in making nylon, the addition of hydrogen cyanide to butadiene also gives 2-methylglutaronitrile (MGN) as a by-product in large quantities. Kirk-Othmer, Encyclopedia of Chemical Technology, Third Edition, Vol. 15, p. 899; U.S. Patent Nos. 3,542,847 and 3,551,474. Hydrogenation of this MGN can then provide 2-methyl-1,5-pentanediamine (MPDA) as a major product.

For example, British Patent No. 1,488,335 issued in 1977 to Dynamit Nobel describes MGN hydrogenation in the prior art to mostly 2-methyl-1,5-pentanediamine (MPDA) and some 3-methylpiperidine, while examples in the '335 patent describe reversed product ratios as its invention. U.S. Patent No. 2,790,804 issued in 1957 to ICI and British Patent No. 2,165,844 issued in 1986 to ICI similarly describe hydrogenation of unsubstituted glutaronitrile to pentanediamine and piperidine. MPDA can also be conveniently prepared by hydrogenation of 2-methyleneglutaronitrile, which is a product of the dimerization of acrylonitrile. British Patent No. 1,164,354 issued in 1969 to Toyo Rayon; U.S. Patent No. 3,225,083 issued in 1965 to Shell; U.S. Patent No. 3,562,311 issued in 1971 to Shell; and U.S. Patent No. 4,422,981 issued in 1983 to Mitsubishi.

Pyridine derivatives, on the other hand, are known to be useful for many purposes. For example, pyridine is valuable as a solvent and as an intermediate for agricultural chemicals. 3-Methylpyridine (beta-picoline) is itself useful as a solvent and as an intermediate for the manufacture of nicotinic acid and nicotinamide, both forms of the pellagra-preventative vitamin. Goe, "Pyridine and Pyridine Derivatives", Kirk-Othmer, Encyclopedia of Chemical Technology, Third Edition, Vol. 19.

In the past, cyclization and dehydrogenation reactions known to produce pyridine and its derivatives have been comprehensively reviewed originally by Brody and Ruby in Volume 1 of Pyridine and Its Derivatives, E. Klingsberg, ed., and most recently by Bailey, Goe and Scriven, in Vol. 5 of the Supplement to Pyridine and Its Derivatives, G. C. Newkome, ed. These reactions have generally been carried out in the gas phase at low to moderate temperatures up to about 400°C and for various times using predominantly precious metal catalysts such as palladium and platinum. For example, British Patent No. 755,534 issued in 1956 to ICI describes the conversion of pentanediamine (PDA) to pyridine in 55% yield using a catalyst of 5% platinum on a silica support at 400°C. This document also reports the conversion of PDA to piperidine using acidic heterogeneous catalysts such as silica, silica-alumina beads and boron phosphate, without the precious metal or any other metal component at 350°C. Other examples include the following:

Netherlands patent application No. 7,005,792 (Deumens, Groen, and Lipsch, 1971 to Stamicarbon; Chem. Abstr., 76, 46099) reports converting PDA to piperidine in high yield using a catalyst of Raney-nickel supported on silica or to various mixtures of piperidine and pyridine using a catalyst of palladium supported on alumina at 125-300°C.

U.S. Patent No. 4,086,237 issued in 1978 to Dynamit Nobel (equivalent to German Patent No. 2,519,529) reports the conversion of MPDA alone or with 3-methylpiperidine to mostly 3-methylpyridine using palladium metal on a silica support at 200-400°C. U.S. Patent No. 4,401,819 issued in 1983 to Rhone-Poulenc reports a similar conversion using a precious metal on a particular macroporous solid silica support at 200-500°C, or more preferred at 250-400°C.

British patent application No. 2,165,844 filed in 1986 by ICI reports the eventual conversion of glutaronitrile to pyridine, perhaps with the preferred isolation of 1,5-pentanediamine as an intermediate, using palladium metal on silica support at 350-400°C.

Collectively, these references show that pentanediamine and its alkyl derivatives have been selectively converted in the past to their piperidine counterparts using catalyst supports alone or in combination with the Group VIII nickel metal, or to admixtures of these piperidines and their pyridine counterparts using various Group VIII precious metals (also called noble metals) including palladium and platinum. This work has suffered from the disadvantage that only these precious metal catalysts have been shown to selectively produce acceptably-high yields of the pyridine compounds such as 3-methylpyridine. Besides their high initial cost, these expensive precious metal catalysts pose added handling problems and cannot be economically used in fluid-bed reactors (which are advantageous for many reasons including their temperature uniformity and ease of catalyst regeneration) because of the catalyst losses that inevitably occur in such processes.

Thus, there has been a growing need and economic driving force for a process useful for the selective conversion of pentanediamine derivatives (such as 2-methyl-1,5-pentanediamine) to their pyridine counterparts (such as 3-methylpyridine) in high yields using effective and readily available catalysts that are inexpensive, that are susceptible of regeneration, and that most preferably can be operated in fluid-bed reactors. The applicants' discovery meets these needs.

### Summary of the Invention

The applicants have discovered a process for the selective production of 3-methylpyridine in high yield directly from 2-methyl-1,5-pentanediamine (MPDA) alone or admixed with 3-methylpiperidine, as in the hydrogenation products of 2-methylglutaronitrile (MGN). This process comprises the step of contacting a vaporized feed stream containing at least the acyclic MPDA compound with a transition metal-oxide catalyst of copper-chromium or of molybdenum at a temperature of about 400-600°C for a time of less than about 30 seconds. This is a significant improvement over prior art processes using Group VIII precious metal catalysts in these reactions.

One embodiment of this process utilizes a fluid-bed reactor for efficiency and ease of operation including separation and recovery of the condensed 3-methylpyridine product and recycling of any 3-methylpiperidine that may be present. In other embodiments, a copper chromite and a molybdenum oxide are the catalysts of choice either unsupported or on a suitable heterogeneous support such as silica, alumina or a combination thereof as in an amorphous or a crystalline zeolite form. In the case of copper chromites, also preferred has been the presence of barium or manganese in its oxide form. Possible additives to the feed stream include water, hydrogen, ammonia, and nitrogen or other inert gases.

The applicants have also discovered that at high-end temperatures of about 500-600°C, additional advantages of significantly-extended catalyst life and activation have been experienced with their preferred catalysts without the need for as frequent regeneration. Contact times of about 10 seconds or less have also shown to be preferred, which lessen the chance of product decomposition at these elevated temperatures while maintaining high levels of conversion and yield of the desired 3-methylpyridine product.

Related objects and advantages of the present invention will be apparent from the following description.

### Description of the Preferred Embodiment

For the purposes of promoting an understanding of the principles of the invention, reference will now be made to the preferred embodiment and specific language will be used to describe the same. It will nevertheless be understood that no limitation of the scope of the invention is thereby intended, such alterations and further modifications in the invention and such further applications of the principles of the invention as described herein being contemplated as would normally occur to one skilled in the art to which the invention relates.

As stated above, the applicants have discovered that a pentanediamine derivative, preferably 2-methyl-1,5-pentanediamine (MPDA), is readily and selectively converted in high yield to its pyridine counterpart, preferably 3-methylpyridine, using improved catalysts made of transition metal oxides not of the Group VIII precious metals used in the prior art. In particular, the applicants' preferred process achieves this selective production of 3-methylpyridine in high yield by contacting a vaporized feed stream containing 2-methyl-1,5-pentanediamine with a transition metal-oxide catalyst predominantly of copper-chromium or molybdenum. This contacting step (and resulting reaction) preferably takes place at a temperature of about 400-600°C and for a time of less than about 30 seconds.

The applicants' effective catalysts have included both supported and unsupported forms. It is appreciated, however, that catalysts supported on a suitable low-cost heterogeneous support such as silica, alumina or silica-alumina in some form can be an economic advantage particularly in a fluid-bed operation. The most effective and preferred catalysts to date have comprised copper chromites (particularly those unsupported and promoted with barium or manganese in oxide form) and molybdenum oxides on a variety of supports.

In this regard, the applicants' use of the term "copper-chromium" defines a class of metal-oxide catalysts containing copper and chromium which may be present in varied valence states and have been subjected to a reducing atmosphere in the presence of hydrogen or other reactants pursuant to one of many different procedures. This term, and these catalysts and their preparations, have long been known in the art as, for example, discussed in the 1937 book by Homer Adkins entitled Reactions of Hydrogen with Organic Compounds over Copper-Chromium Oxide and Nickel Catalysts (and particularly pages 11-14) which is incorporated herein by reference as to all matters relevant and material to this application. Similarly, the term "copper chromite" is of more contemporary vintage and defines the class of catalysts comprising copper and chromium in various oxide forms after being calcined according to standard and known procedures. Several copper chromites are commercially available, for example, as identified in the specific Examples below.

The applicants' use of the term "effective" relates to the catalyst's ability to selectively produce the desired 3-methylpyridine product in high yields under the preferred reaction steps and conditions as defined herein. Various of the experimental results achieved by the applicants to date are set forth in the specific Examples and Table below. However, in view of the number of variables present, an "effective" or "high" yield of 3-methylpyridine under these circumstances constitutes one that is commercially significant. This is preferably one that approaches a net yield of about 50% or more 3-methylpyridine based on a conversion approaching 100% of the original organic feed stream. Alternatively, it is also preferred that by-products be limited principally to 3-methylpiperidine in a comparative yield of about 1:2 or less relative to the 3-methylpyridine produced. This 3-methylpiperidine can then be recycled back through the feed stream to make more of the desired pyridine product.

Methods for the preparation of the applicants' catalysts are well known in the art. The preferred method to date in the case of an unsupported catalyst has been for the transition metal salt to be precipitated and then decomposed to the desired oxide by heat. For a supported catalyst, a salt that is soluble in water, such as ammonium molybdate or various nitrates, has been first absorbed on the desired support and then decomposed to the desired oxide by heat (also known as calcining). An alternative method is to form the desired transition metal oxide as an ion-exchanged form of a zeolite, and then to calcine the resulting zeolite salt to form the desired catalyst. These and other methods known to those of ordinary skill in this art can be used in preparing the catalysts of the applicants' preferred embodiments. Suitable catalysts are also commercially available as in the case of an unsupported copper chromite material marketed by The Harshaw/Filtrol Partnership (now Engelhard Corporation) of Cleveland, Ohio. Regardless of their source, however, these catalysts can be prepared or purchased in many usable sizes and shapes such as pellets, extrusions or spheres for fixed-bed use or as powders or microspheroidal materials for fluid-bed use. These and other physical factors involved in catalyst selection, preparation and handling vary with the specific equipment, conditions and reaction selected, and are well within the ordinary skill of those in this field.

The applicants' reactions have been preferably carried out in the usual fashion of continuous gas-phase reactions of this type, in which the reactants are vaporized and this feed stream then passed in contact with the catalyst which is maintained at the desired temperature. In this way, the vaporized reactants are conducted over the catalyst to produce a suitable contact time for the reaction to take place. This contact time may be seen as the time required to achieve a desired or maximum conversion which is often expressed as a percentage of the original reactants passed. The preferred contact time in a particular reaction must be found by trial and error under the specific circumstances involved, unless prior comparative data is available.

The applicants have found that contact times of about 30 seconds or less are preferred in their work to date. Contact times of about 10 seconds or less have proven even more desirable. In fact, experiments have shown that minimizing the contact time is preferred and that conversions approaching 100% of the reactants used and net yields of 3-methylpyridine approaching about 50% or more of these conversions have still been achieved. Significantly longer contact times may require specially designed equipment, and can result in product decomposition or other unwanted by-products at the elevated temperatures involved.

For example, the applicants' reactions have been carried out at preferred temperatures in the range from about 400-600°C. These temperatures have maximized the conversions achieved with a preference to the desired 3-methylpyridine product. Even more preferred have been temperatures of about 500-600°C, with about 550°C being most preferred to date for maximizing conversion and for other advantages discussed below. In this regard, each reaction must be examined on its own to determine optimum conditions, including temperature, under given circumstances.

The applicants' reactions have proven suitable for fixed-bed or fluid-bed operation. Fixed-bed reactors in this field are well documented both in practice and in the literature. The same is true of fluid-bed reactors, although more variables exist. For example, the feed rates of the vapor reactants are chosen to give sufficient fluidization of the catalyst bed. These are usually at a superficial velocity between about 0.25 ft/sec (0.08 m/sec) and 3.0 ft/sec (0.9 m/sec), although lower or higher velocities may be chosen in given circumstances. The reaction products are then collected by condensation and individual products are separated and recovered as desired, frequently by distillation means. As noted below, if the process yields a mixture of the piperidine and pyridine derivatives, one alternative is to subject the mixture to further catalytic reaction to dehydrogenate the remaining piperidine material. Another alternative is to first isolate the pyridine product and then to recycle only the piperidine component back through the reactor. In any case, the general construction and operation of a fluid-bed or a fixed-bed reactor are no different for the applicants' processes than for other reactions for which they are used. Reference can thus be made to available literature or other sources in this area as to the specific establishment and operation of such reactors, the same being well within the skill of those practiced in this art.

As for the starting materials used, the applicants' preferred feed stream needs only to contain an amount of 2-methyl-1,5-pentanediamine (MPDA) as a reactant for the process. This acyclic compound is vaporized and passed in contact with the heated catalyst bed to bring about the reaction. Other materials may also be present in the feed stream as long as they do not interfere significantly with the selective production of 3-methylpyridine in high yields as previously described.

For example, since pentanediamines have been known to cyclize producing a mixture of pyridine and piperidine derivatives under prior art conditions, one alternative has been the inclusion of the corresponding 3-methylpiperidine compound in the feedstock without detracting from the advantages achieved with the applicants' preferred processes. The 3-methylpiperidine present has simply dehydrogenated thereby producing even more of the desired 3-methylpyridine product. Since pentanediamines have been produced by hydrogenation of a dinitrile such as 2-methylglutaronitrile (MGN), which is also known to produce cyclized compounds such as piperidines as by-products, a useful feedstock for the applicants' reactions has been a mixture of the pentanediamine and piperidine derivatives produced by the hydrogenation of this MGN material. Separation of these MGN hydrogenation products as feed components has been unnecessary under these circumstances, which is a substantial time and cost savings over many prior art processes in this area.

Additional materials have proven suitable for inclusion in the applicants' reaction feed stream as well. One such additive has been hydrogen, which when used has shown some advantage in a molar ratio of about 1:1 or more hydrogen to organic in the feed stream. Since hydrogen is not consumed, but rather generated in the reaction, it is advantageous in a commercial application to recycle this hydrogen. It may also be possible to obtain hydrogen initially as a by-product of some other process in the plant.

A second possible additive has been water which may be supplied in the form of steam. This additive has been used with some advantage in experiments to date in a molar ratio of about 5:1 or more water to organic in the feed stream. It should be recognized, however, that any water remaining in the product mixture must be later separated out and that the failure to account for this water can reflect as a lower material balance for the reaction. Evidence of this is found in the Table below.

Still other possible additives have included nitrogen (or other inert gases) and ammonia. Nitrogen has been used as a diluent or carrier gas particularly in reactions with small amounts of organic component in the feed stream. Ammonia has been used in other experiments, both additives being in molar ratios of about 5:1 or more to the organic component. Since ammonia is a reaction product similar to hydrogen, however, it must be recovered in any commercial application and possibly purged for use elsewhere or destroyed.

A recognized problem with catalysts in this area has been that their activities gradually decrease over time. With most catalysts regeneration is possible, for example, by heating in the presence of air or some other oxygen-containing gas. See Charles L. Thomas, Catalytic Processes and Proven Catalysts, pp. 11-14 (1970). This may be followed by passing hydrogen over the hot catalyst before returning it to contact with a further reactant stream. This need for periodic regeneration encourages the use of fluid beds for such reactions, which beds are capable of being regenerated either in total at certain intervals or in part by the catalyst being continuously or intermittently circulated to a second reaction vessel in which regeneration takes place. Such reactors are commonly used in industry for reactions such as the catalytic cracking of petroleum and in pyridine synthesis.

Another aspect of the applicants' invention has been the discovery that their preferred catalysts have been able to retain acceptable activity levels for significantly longer times than are common in this art. This has occurred at the upper level of preferred temperatures in the range of about 500-600°C, with about 550°C being most preferred to date. This is substantially above any temperature known to have been taught or suggested in the art as being acceptable for this type of reaction. While quantitative analysis in this area is difficult, the applicants have found that repeated runs with their preferred catalysts at these temperatures have shown fairly constant and acceptable conversions and yields without intervening regeneration. This is a significant advantage in catalyst life and in the elimination of the downtime normally involved in regeneration.

While the invention has been described in detail in the foregoing paragraphs, the same is to be considered as illustrative and not restrictive in character. It is understood that only the preferred embodiments have been described and that all changes and modifications that come within the spirit of the invention are desired to be protected.

In this regard, some specific Examples and a Table follow which reflect experimental runs performed by the applicants using their preferred catalysts and processes described above. In these, reference is made to 2-methyl-1,5-pentanediamine as "MPDA," to 3-methylpiperidine as "MePip" and to 3-methylpyridine as "Beta." "Conversion" is expressed as a percentage calculated by dividing moles of organic compound reacted by moles of organic compound fed into the reactor in the feed stream. "Gross yield" is expressed as a percentage calculated by dividing moles of specific product obtained by moles of organic compound fed into the reactor in the feed stream. "Net yield" is expressed as a percentage calculated by dividing moles of product obtained by moles of organic compound reacted. "Contact times" for the reactions were all in the range of about 4-10 seconds, unless otherwise specified, and minimal superficial velocities ("Sup. Vel.") were observed to maintain fluidization of the catalyst bed.

In all but Example 28, a fluid-bed reactor was used. This reactor consisted of a 1.6-inch (4.1-cm) ID x 5-foot (1.5-m) 316 stainless steel pipe with a gas furnace covering the lower 3 feet (0.9 meters) and a 5-inch (13-cm) ID disengaging bell equipped with a filter at the top. The catalyst charge was generally 750 ml and the catalyst particles were of a size suitable for fluidization, generally in the range of about 20-850 micrometers (or microns). The key in this regard is to be able to fluidize the particle bed, and the equipment used will often dictate the preferred particle size for this purpose. In this regard, the feed vaporizer used was an electrically heated 0.75 inch (1.9-cm) x 26-inch (66-cm) stainless steel pipe. Vaporized feed was introduced into the bed by means of a perforated tube sprayer. Gases were preheated by passage through an electrically heated 0.25-inch (0.64-cm) x 20-inch (51-cm) stainless steel pipe. Preheated gases were introduced into the bed by means of a distributor plate.

### EXAMPLE 1

80 g of MPDA/hour and 95 g of water/hour were fed from a calibrated blowpot into the fluid-bed reactor containing 625 ml (1107g) of Harshaw copper chromite catalyst (#Cu-1107T, containing 33% CuO, 37% Cr₂O₃ and 7% BaO as listed active components). The MPDA used was obtained commercially under the trademark "DYTEK-A" marketed by the E. I. duPont de Nemours & Company, and was used in all the Examples below unless otherwise specified. The catalyst was originally in tablet form, and had been crushed to pass through 20-mesh (850-micrometer) screen. The feed was vaporized and heated to 360°C. At the same time, 500L of hydrogen/hour (mole ratio H₂/MPDA = 30) were heated to 160°C and passed into the reactor. The reactor temperature was maintained at 395°C. The product was collected by condensation in a 6-foot (1.8-m) water cooled condensor followed by a coil of tubing submerged in ice water. The combined product from the first 30-minute run was analyzed by gas chromatography, showing 100% conversion with 17% net yield of MePip and 80% net yield of Beta which was a significantly high result even compared to prior art processes using expensive precious metal-oxide catalysts. The combined product from the second 30-minute run showed 100% conversion with 43% net yield of Beta which was still acceptably high. However, the net yield of MePip increased to 51% showing catalyst deactivation as is common and reported in the art at the temperature of this reaction. The MePip from each run was retained and recycled in later feed streams to produce more of the desired Beta product.

### EXAMPLE 2

The reaction was carried out as in Example 1, but 600 ml (822g) of copper chromite obtained from Alfa Chemicals (#11843, containing 78% CuO and 20% Cr203 as listed active components) was crushed to pass through a 20-mesh (850-micrometer) screen for use as the catalyst and no water was added to the feed. Thus, a feed of 402g of MPDA/hour and 1563 L of hydrogen/hour (mole ratio H2/MPDA = 18) at a reaction temperature of 400°C resulted in 98% conversion with 10% net yield of MePip and an effective net yield of 45% of Beta in the first 30 minutes of the run and in 92% conversion with 6% net yield of MePip and a much lower 19% net yield of Beta in the second 30 minutes. The catalyst was then regenerated in the usual manner, as follows: The flows of organic and hydrogen were stopped, the system was purged with nitrogen for about 5 minutes, and the catalyst was fluidized with approximately 400 L of air/hour. The bed temperature was raised to and maintained at about 600°C for about 30 minutes, and then reduced to 440°C for the next runs in which 493g of MPDA/hour and 1346 L of hydrogen/hour (mole ratio H2/MPDA - 13) resulted in 97% conversion with 9% net yield of MePip and 40% net yield of Beta in the first 30-minute run and 92% conversion with 10% net yield of MePip and 26% net yield of Beta in the second 30 minutes.

### EXAMPLE 3

The reaction was carried out as in Example 1, but 750 ml (535g) of MoO₃ on silica-alumina (containing 10% MoO₃, 78% silica and 12% alumina) was the catalyst, nitrogen was used in place of hydrogen and the reaction temperature was raised to 545°C. The catalyst was prepared by a standard procedure in which 600g of silica-alumina was impregnated with a 480 mL solution containing 78g of molybdic acid in concentrated ammonium hydroxide. The catalyst was allowed to dry overnight, and was then calcined at 500°C. Thereafter, 157g of MPDA/hour, 174g of water/hour and 209 L of nitrogen/hour (mole ratio N₂/MPDA - 6) resulted in 100% conversion with 0.3% net yield of MePip and 97% net yield of Beta in the first 30-minute run and 100% conversion with 0.4% Ret yield of MePip and 95% net yield of Beta in the second 30 minutes. Both of these results were extremely high and selective yields of Beta and significant improvements over prior art processes.

### EXAMPLE 4

The reaction was carried out as in Example 3 using 525 ml (358g) of the same MoO₃ on silica-alumina catalyst at a reaction temperature of 553°C. Thereafter, consecutive runs of 216g of MPDA/hour, 216g of water/hour and 210 L of nitrogen/hour (mole ratio N₂/MPDA = 5) resulted in 0.1% net yield of MePip and 73% net yield of Beta in the first-hour run, 0.5% net yield of MePip and 99% net yield of Beta in the second hour, 0.7% net yield of MePip and 86% net yield of Beta in the third hour, 1.4% net yield of MePip and 81% net yield of Beta in the fourth hour, and 1.6% net yield of MePip and 81% net yield of Beta in the fifth hour. Conversions were 100% throughout the runs. These significantly selective and high yields of Beta were achieved without regeneration of the catalyst and with no indication of catalyst deactivation over time as has been common in this area. These superior results were also in contrast to those obtained using the same components, but at a temperature of 400°C and with hydrogen in place of nitrogen. In those runs the initial net yield of Beta was 34%, but it decreased to 25% by the end of the first hour and no further runs were made as the trend was obvious toward decreasing and unacceptable yields without regeneration.

### EXAMPLES 5-24 (See Table. p. 20-A)

The reactions in these Examples were carried out using the equipment and procedures as described previously in Example 1. Catalyst components were obtained commercially, and their supported forms prepared where needed using standard impregnation techniques such as described in Example 3. The organic phase of the feed stream in each Example was MPDA ("DYTEK-A" from duPont) alone, except as noted in the Table. Each Example was the first 30-minute run for the indicated catalyst, except for the following: While Example 8 using a mixed MPDA-MePip feed stream at 406°C was the first 30-minute run, Example 19 was the second 30-minute run using similar components at 552°C and showed that continued selective and high yields of Beta were achieved without catalyst regeneration. Examples 23(a)-23(g) were consecutive and continuous runs at about 550°C using the same batch of catalyst in which runs (a)-(b) were 30 minutes long and runs (c)-(g) were 60 minutes long. These runs further evidenced the effective and relatively constant yields of Beta that applicants achieved over extended periods at this temperture without the necessity of catalyst regeneration.

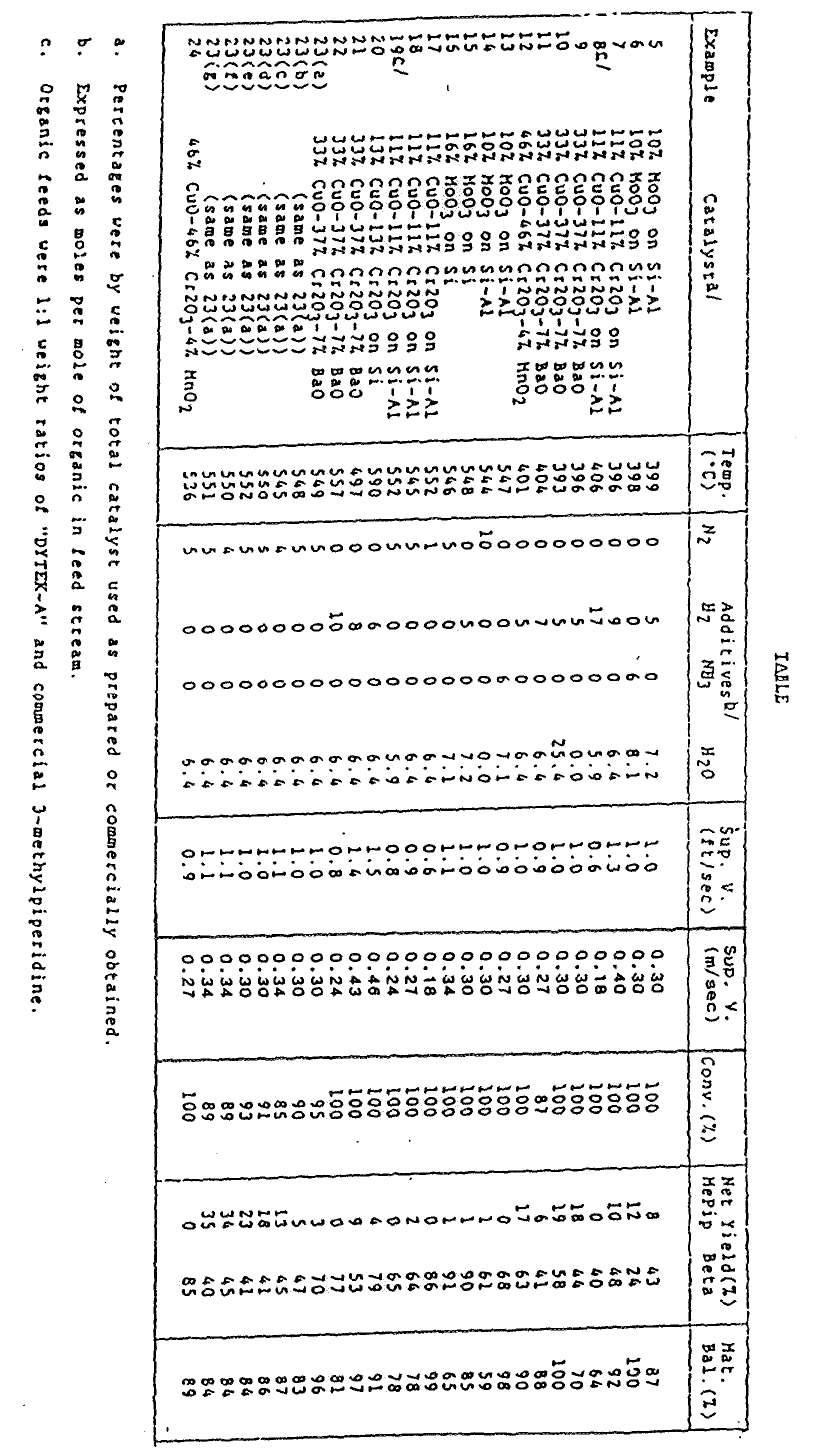

### EXAMPLE 25

In this Example, a fixed-bed reactor was used. It comprised a 1-inch (2.5-cm) ID x 3-foot (91-cm) 316 stainless steel pipe covered with a sodium-filled jacket, covered in turn with a gas furnace. The catalyst charge was 250 ml (401g) of Harshaw copper chromite #Cu-1107T as used in Example 1. The form of the catalyst was 0.125-inch (0.32-cm) x 0.125-inch (0.32-cm) cylindrical pellets. 24g of MPDA/hour and 26g of water/hour were fed from a single-stroke piston pump, vaporized and heated to 240°C in an electrically heated 0.5-inch (1.3-cm) x 1-foot (30-cm) stainless steel pipe and passed downward through the reactor. The reactor temperature was maintained at 552°C. The product was collected by condensation in a coil of tubing submerged in ice water. The combined product from the 1-hour run was analyzed as in Example 1, showing 94% conversion with no MePip and 97% net yield of Beta.

### EXAMPLE 26

This reaction was carried out as in Example 3 using 760 mL (700g) of MoO₃ on an equilibrium cracking catalyst (containing 6% MoO₃) which was prepared in the conventional manner. The organic feed was from the hydrogenation of MGN, comprising a mixture of 42% MePip, 51% MPDA and 7% other organic by-products. Thereafter, a feed of 220 g of the MGN hydrogenation products/hour, 230 g of water/hour and 209 L of nitrogen/hour at a reaction temperature of 550°C resulted in 99% conversion of the feed with 76% net yield of Beta in the first 30-minute run and 100% conversion of the feed with 83% net yield of Beta in the second 30-minute run. Only traces of MePip were seen in the recovered product from these runs.

## Claims

1. A process for the selective production of 3-methylpyridine in high yield, comprising the step of:
contacting a vaporized feed stream containing 2-methyl-1,5-pentanediamine with a transition metal-oxide catalyst of copper-chromium or molybdenum, said contacting being at a temperature of about 400-600°C for a time of less than about 30 seconds.

2. The process of claim 1 in which the feed stream also contains 3-methylpiperidine.

3. The process of claim 2 in which the feed stream contains the hydrogenation products of 2-methylglutaronitrile.

4. The process of claim 1 in which said contacting is in a fluid-bed reactor.

5. The process of claim 1, 2 or 4 in which the catalyst comprises a copper chromite or a molybdenum oxide.

6. The process of claim 1, 2 or 4 additionally comprising the steps of:
separating and recovering the 3-methylpyridine product after said contacting.

7. The process of claim 6 additionally comprising the steps of:
initially charging a reactor with an amount of the catalyst, said contacting including passing the vaporized feed stream through the charged and heated reactor, said separating and recovering including condensing the product stream exiting the reactor and isolating the 3-methylpyridine product by distillation.

8. The process of claim 7 additionally comprising the steps of:
isolating any 3-methylpiperidine product in the stream exiting the reactor also by distillation and recycling the same to said contacting step.

9. The process of claim 8 in which said isolated 3-methylpiperidine product is present in a comparative yield of about 1:2 or less relative to said recovered 3-methylpyridine product.

10. The process of claim 6 in which the catalyst is on a suitable heterogeneous support such as silica, alumina or a combination thereof.

11. The process of claim 6 in which the feed stream also contains water.

12. The process of claim 11 in which the water is present in a molar ratio of about 5:1 or more water to organic in the feed stream.

13. The process of claim 6 in which the feed stream also contains hydrogen.

14. The process of claim 13 in which the hydrogen is present in a molar ratio of about 1:1 or more hydrogen to organic in the feed stream.

15. The process of claim 6 in which the feed stream also contains ammonia.

16. The process of claim 15 in which the ammonia is present in a molar ratio of about 1:1 or more ammonia to organic in the feed stream.

17. The process of claim 6 in which the feed stream also contains nitrogen or some other inert gas.

18. The process of claim 17 in which the nitrogen or other inert gas is present in a molar ratio of about 1:1 or more compared to organic in the feed stream.

19. The process of claim 6 in which the catalyst comprises a copper chromite or a molybdenum oxide.

20. The process of claim 19 in which the catalyst comprises copper chromite.

21. The process of claim 20 in which the copper chromite catalyst also contains an oxide of barium or manganese.

22. The process of claim 19 in which the catalyst comprises molybdenum oxide.

23. The process of claim 19 in which said recovered 3-methylpyridine product is present in an effective yield approaching about 50% or more based on a conversion approaching 100% of the feed stream after said contacting.

24. The process of claim 19 in which said contacting is at a temperature of about 500-600°C.

25. The process of claim 24 in which said contacting is at a temperature of about 550°C.

26. The process of claim 24 in which said contacting is for a time less than about 10 seconds.

27. The process of claim 26 whereby said contacting, separating and recovering steps can be repeated with new or recycled feed streams and without regenerating the catalyst while still recovering effective yields of the 3-methylpyridine product.

28. The process of claim 26 additionally comprising repeating runs of said contacting, separating and recovering steps with new or recycled feed streams and without regenerating the catalyst.

29. The process of claim 28 in which said recovered 3-methylpyridine product is present in comparative yields of about 2:1 or more relative to any 3-methylpiperidine product from said runs.

30. The process of claim 29 in which said recovered 3-methylpyridine product is present in effective yields approaching about 50% or more based on conversions approaching 100% of the feed streams in said runs.

## Patentansprüche

1. Verfahren zur selektiven Erzeugung von 3-Methylpyridin mit hoher Ausbeute, das den folgenden Schritt umfaßt:
Inkontaktbringen eines verdampften Zufuhrstroms, der 2-Methyl-1,5-pentandiamin enthält, mit einem Übergangsmetalloxid-Katalysator aus Kupfer-Chrom oder Molybdän, wobei das Inkontaktbringen bei einer Temperatur von etwa 400°C bis 600°C während einer Zeitdauer von weniger als etwa 30 Sekunden erfolgt.

2. Verfahren nach Anspruch 1, bei dem der Zufuhrstrom außerdem 3-Methylpiperidin enthält.

3. Verfahren nach Anspruch 2, bei dem der Zufuhrstrom die Hydrierungsprodukte von 2-Methylglutaronitril enthält.

4. Verfahren nach Anspruch 1, bei dem das Inkontaktbringen in einem Fließbettreaktor stattfindet.

5. Verfahren nach Anspruch 1, 2 oder 4, bei dem der Katalysator ein Kupferchromit oder ein Molybdänoxid beinhaltet.

6. Verfahren nach Anspruch 1, 2 oder 4, das zusätzlich die folgenden Schritte umfaßt:
Abtrennen und Gewinnen des 3-Methylpyridin-Produkts nach dem Inkontaktbringen.

7. Verfahren nach Anspruch 6, das zusätzlich die folgenden Schritte umfaßt:
anfängliches Beschicken eines Reaktors mit einer Menge des Katalysators, wobei das Inkontaktbringen ein Hindurchleiten des verdampften Zufuhrstroms durch den beschickten und geheizten Reaktor beinhaltet, wobei das Abtrennen und Gewinnen ein Kondensieren des Produktstromes, der den Reaktor verläßt, sowie ein Isolieren des 3-Methylpyridin-Produkts durch Destillation beinhaltet.

8. Verfahren nach Anspruch 7, das zusätzlich die folgenden Schritte umfaßt:
Isolieren jeglichen 3-Methylpiperidin-Produkts in dem Strom, der den Reaktor verläßt, ebenfalls durch Destillation und Rückführen desselben zu dem Inkontaktbringungsschritt.

9. Verfahren nach Anspruch 8, bei dem das isolierte 3-Methylpiperidin-Produkt in einer relativen Ausbeute von etwa 1:2 oder weniger relativ zu dem gewonnenen 3-Methylpyridin-Produkt vorliegt.

10. Verfahren nach Anspruch 6, bei dem der Katalysator auf einem geeigneten heterogenen Träger, wie Siliciumdioxid, Aluminiumoxid oder einer Kombination derselben, vorliegt.

11. Verfahren nach Anspruch 6, bei dem der Zufuhrstrom außerdem Wasser enthält.

12. Verfahren nach Anspruch 11, bei dem das Wasser in einem molaren Verhältnis von etwa 5:1 oder mehr Wasser zu organischer Verbindung in dem Zufuhrstrom vorliegt.

13. Verfahren nach Anspruch 6, bei dem der Zufuhrstrom außerdem Wasserstoff enthält.

14. Verfahren nach Anspruch 13, bei dem der Wasserstoff in einem molaren Verhältnis von etwa 1:1 oder mehr Wasserstoff zu organischer Verbindung in dem Zufuhrstrom vorliegt.

15. Verfahren nach Anspruch 6, bei dem der Zufuhrstrom außerdem Ammoniak enthält.

16. Verfahren nach Anspruch 15, bei dem der Ammoniak in einem molaren Verhältnis von etwa 1:1 oder mehr Ammoniak zu organischer Verbindung in dem Zufuhrstrom vorliegt.

17. Verfahren nach Anspruch 6, bei dem der Zufuhrstrom außerdem Stickstoff oder irgendein anderes inertes Gas enthält.

18. Verfahren nach Anspruch 17, bei dem der Stickstoff oder das andere inerte Gas in einem molaren Verhältnis von etwa 1:1 oder mehr im Vergleich zu der organischen Verbindung in dem Zufuhrstrom vorliegt.

19. Verfahren nach Anspruch 6, bei dem der Katalysator ein Kupferchromit oder ein Molybdänoxid beinhaltet.

20. Verfahren nach Anspruch 19, bei dem der Katalysator Kupferchromit beinhaltet.

21. Verfahren nach Anspruch 20, bei dem der Kupferchromit-Katalysator außerdem ein Oxid von Barium oder Mangan enthält.

22. Verfahren nach Anspruch 19, bei dem der Katalysator Molybdänoxid beinhaltet.

23. Verfahren nach Anspruch 19, bei dem das gewonnene 3-Methylpyridin-Produkt in einer effektiven Ausbeute vorliegt, die nahe bei etwa 50% oder mehr liegt, basierend auf einer nahe bei 100% liegenden Umwandlung des Zufuhrstroms nach dem Inkontaktbringen.

24. Verfahren nach Anspruch 19, bei dem das Inkontaktbringen bei einer Temperatur von etwa 500°C bis 600°C erfolgt.

25. Verfahren nach Anspruch 24, bei dem das Inkontaktbringen bei einer Temperatur von etwa 550°C erfolgt.

26. Verfahren nach Anspruch 24, bei dem das Inkontaktbringen während einer Zeitdauer von weniger als etwa 10 Sekunden erfolgt.

27. Verfahren nach Anspruch 26, bei dem die Inkontaktbringungs-, Abtrenn- und Gewinnungsschritte mit neuen oder rückgeführten Zufuhrströmen und ohne Regenerierung des Katalysators wiederholt werden können, während weiterhin effektive Ausbeuten des 3-Methylpyridin-Produkts gewonnen werden.

28. Verfahren nach Anspruch 26, das zusätzlich Wiederholungsdurchläufe der Inkontaktbringungs-, Abtrenn- und Gewinnungsschritte mit neuen oder rückgeführten Zufuhrströmen und ohne Regenerieren des Katalysators umfaßt.

29. Verfahren nach Anspruch 28, bei dem das gewonnene 3-Methylpyridin-Produkt in relativen Ausbeuten von etwa 2:1 oder mehr relativ zu jeglichem 3-Methylpiperidin-Produkt aus den Durchläufen vorliegt.

30. Verfahren nach Anspruch 29, bei dem das gewonnene 3-Methylpyridin-Produkt in effektiven Ausbeuten vorliegt, die nahe bei etwa 50% oder mehr liegen, basierend auf nahe bei 100% liegenden Umwandlungen der Zufuhrströme in den Durchläufen.

## Revendications

1. Procédé de production sélective de 3-méthylpyridine avec un rendement élevé qui comprend l'étape de mise en contact d'un courant d'alimentation vaporisé contenant de la 2-méthyl-1,5-pentanediamine avec un catalyseur à base d'oxyde de métal de transition de cuivre-chrome ou de molybdène, cette mise en contact étant à une température environ de 400-600°C pendant un temps de moins environ de 30 secondes.

2. Procédé selon la revendication 1 dans lequel le courant d'alimentation contient également de la 3-méthylpipéridine.

3. Procédé selon la revendication 2 dans lequel le courant d'alimentation contient les produits d'hydrogénation du 2-méthylglutaronitrile.

4. Procédé selon la revendication 1 dans lequel ladite mise en contact est dans un réacteur à lit fluidisé.

5. Procédé selon les revendications 1, 2 ou 4, dans lequel le catalyseur comprend un chromite de cuivre ou un oxyde de molybdène.

6. Procédé selon les revendications 1, 2 ou 4, comprenant en supplément les étapes qui consistent en la séparation et la récupération du produit 3-méthylpyridinique après la dite mise en contact.

7. Procédé selon la revendication 6, comprenant en supplément les étapes qui consistent en le chargement initial d'un réacteur avec une quantité de catalyseur, ladite mise en contact comprenant le passage du courant d'alimentation à travers le réacteur chargé et chauffé, lesdites séparations et récupérations comprenant le fait de condenser le courant de produit quittant le réacteur et le fait d'isoler le produit 3-méthylpyridinique par distillation.

8. Procédé selon la revendication 7, comprenant en supplément les étapes qui consistent dans le fait d'isoler tout produit 3-méthylpiperidine dans le courant quittant le réacteur aussi par distillation et recyclage de celui-ci vers ladite étape de mise en contact.

9. Procédé selon la revendication 8, dans lequel ledit produit 3-méthylpiperidine isolé est présent en un rendement comparatif environ de 1:2 ou moins par rapport au dit produit 3-méthylpyridine récupéré.

10. Procédé selon la revendication 6 dans lequel le catalyseur est sur un support approprié hétérogène, tel que de la silice, de l'alumine ou une combinaison de ceux-ci.

11. Procédé selon la revendication 6, dans lequel le courant d'alimentation contient aussi de l'eau.

12. Procédé selon la revendication 11, dans lequel l'eau est présente en un rapport molaire environ de 5:1 ou plus d'eau, au produit organique dans le courant d'alimentation.

13. Procédé selon la revendication 6, dans lequel le courant d'alimentation contient également de l' hydrogène.

14. Procédé selon la revendication 13, dans lequel l'hydrogène est présent en un rapport molaire environ de 1:1 ou plus d'hydrogène au produit organique dans le courant d'alimentation.

15. Procédé selon la revendication 6, dans lequel le courant d'alimentation contient aussi de l'ammoniaque.

16. Procédé selon la revendication 15 dans lequel l'ammoniaque est présente dans un rapport molaire environ de 1:1 ou plus, d'ammoniaque au produit organique dans le courant d'alimentation.

17. Procédé selon la revendication 6 dans lequel le courant d'alimentation contient aussi de l'azote ou d'autres gaz inertes.

18. Procédé selon la revendication 17 dans lequel l'azote ou un autre gaz inerte est présent dans le courant d'alimentation dans un rapport molaire environ de 1:1 ou plus, comparé au produit organique.

19. Procédé selon la revendication 6 dans lequel le catalyseur contient un chromite de cuivre ou un oxyde de molybdène.

20. Procédé selon la revendication 19 dans lequel le catalyseur contient du chromite de cuivre.

21. Procédé selon la revendication 20 dans lequel le catalyseur à base de chromite de cuivre contient aussi un oxyde de baryum ou de manganèse.

22. Procédé selon la revendication 19 dans lequel le catalyseur contient de l'oxyde de molybdène.

23. Procédé selon la revendication 19 dans lequel ledit produit 3-méthylpyridine récupéré est présent avec un rendement effectif approchant environ 50 % ou plus, basé sur une conversion approchant 100 % du courant d'alimentation après ladite mise en contact.

24. Procédé selon la revendication 19 dans lequel ladite mise en contact est effectuée à une température environ de 500-600°C.

25. Procédé selon la revendication 24 dans lequel ladite mise en contact est effectuée à une température environ de 550°C.

26. Procédé selon la revendication 24 dans lequel ladite mise en contact est effectuée pendant une durée de moins de 10 secondes environ.

27. Procédé selon la revendication 26 dans lequel lesdites étapes de mise en contact, de séparation et de récupération peuvent être répétées avec de nouveaux courants ou des courants recyclés d'alimentation et sans avoir à régénérer le catalyseur tout en récupérant encore des rendements efficaces en produit 3-méthylpyridine.

28. Procédé selon la revendication 26 comprenant en supplément des séries répétées desdites étapes de mise en contact, de séparation et de récupération avec des courants d'alimentation nouveaux ou recyclés et sans régénération du catalyseur.

29. Procédé selon la revendication 28 dans lequel ledit produit 3-méthylpyridine récupéré est présent avec des rendements comparatifs environ de 2:1 ou plus, par rapport à tout produit 3-méthylpipéridine provenant desdites séries.

30. Procédé selon la revendication 29 dans lequel ledit produit 3-méthylpyridine récupéré est présent avec des rendements effectifs approchant environ 50 % ou plus, basé sur des conversions approchant 100 % des courants d'alimentation dans lesdites séries.
